Europäisches Patentamt

European Patent Office

Office européen des brevets

(11) Publication number: **0 241 209**
**A1**

(12) **EUROPEAN PATENT APPLICATION**

(21) Application number: **87302836.9**

(22) Date of filing: **01.04.87**

(51) Int. Cl.³: **C 12 N 15/00**
**C 12 N 9/64, A 61 K 37/54**

(30) Priority: **02.04.86 GB 8608019**
**21.02.87 GB 8704078**

(43) Date of publication of application:
**14.10.87 Bulletin 87/42**

(84) Designated Contracting States:
**BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **Browne, Michael Joseph Beecham**
**Pharmaceuticals**
**Great Burgh Yew Tree Bottom Road**
**Epsom Surrey KT18 5XQ(GB)**

(72) Inventor: **Robinson, Jeffrey Hugh Beecham**
**Pharmaceuticals**
**Great Burgh Yew Tree Bottom Road**
**Epsom Surrey KT18 5XQ(GB)**

(74) Representative: **Valentine, Jill Barbara et al,**
**Beecham Pharmaceuticals Patents & Trade Marks Dept.**
**Great Burgh Yew Tree Bottom Road**
**Epsom Surrey KT18 5XQ(GB)**

(54) Modified enzyme.

(57) A fibrinolytically active tissue-type plasminogen activator which has been modified:
    (a) in the region of the growth factor domain, and
    (b) to provide an amino acid without a positively charged side chain in place of lysine at position 277.

EP 0 241 209 A1

- 1 -

B2055/B2266

TITLE MODIFIED
see front page
Novel Compounds

The present invention relates to a modified fibrinolytic enzyme, in particular modified tissue-type plasminogen activator, its preparation, pharmaceutical compositions containing it and its use in the treatment of thrombotic disease.

The sequence of amino acids making up the enzyme tissue-type plasminogen activator (t-PA) and the nucleotide sequence for the cDNA which codes for t-PA are known (see Pennica et al, 1983; Nature, 301, 214). Tissue-type plasminogen activator is known to have fibrinolytic activity.

It has been shown (Bányai, L. et al, 1983; FEBS Lett., 163, 37)that a part of the t-PA enzyme shows structural homology with human and murine epidermal growth factors. This region from amino acid residues 44 to 91 has been termed the ''growth factor domain''. The genetic information which codes for the major part of this domain, residues 51 to 86 inclusive, and partially codes for residues 50 and 87, lies on a single exon (Ny, T. et al, 1984; Proc. Nat. Acad. Sci. U.S.A., 81, 5355).

EP-A-0 207 589 discloses t-PA modified in the region of the growth factor domain, in particular by the deletion of amino acid residues 51 to 87 inclusive of native t-PA. EP-A-0 201 153 discloses t-PA comprising an amino acid without a charged side chain in place of lysine at position 277, in particular isoleucine.

- 2 -

The applicants have now identified modified forms of the t-PA enzyme which retain fibrinolytic activity.

According to the present invention there is provided a fibrinolytically active tissue-type plasminogen activator which has been modified:

(a)  in the region of the growth factor domain, and

(b)  to provide an amino acid without a positively charged side chain in place of lysine at position 277.

Suitable growth factor domain modifications (modification (a)) may include removal or deletion of certain amino acid residues, or replacement of one or more amino acid residues with different amino acid residues.

In a preferred aspect, the modification (a) comprises the deletion of all or part of the growth factor domain.

In another preferred aspect, the modification (a) occurs in the region from amino acid residues 51 to 87 inclusive, in particular by deletion of that region.

In another preferred aspect, the modification (b) provides an amino acid without a charged side chain in place of lysine at position 277.

In a further preferred aspect, modification (b) is such that the amino acid without a positively charged side chain at position 277 is isoleucine.

As used herein, the term tissue-type plasminogen activator denotes a plasminogen activator of the group

- 3 -

having the immunological properties defined for t-PA at the XXVIII Meeting of the International Committee on Thrombosis and Haemostasis, Bergamo, Italy, 27 July 1982.

The amino acid sequence of various forms of t-PA are known. The abovementioned Nature 1983 reference discloses the sequence for the L-chain and the mature S-chain forms of t-PA, also discussed by Vehar et.al., Biotechnology, 1984, 2, 1051-7 in which the processing of initially formed t-PA by removal of a pro-sequence to give the S-chain form is reported. Pohl et.al., FEBS letters, 1984, Vol. 168 No.1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue at position -3 and U-chain t-PA has an N-terminal valine at position 4. It is understood that the tissue-type plasminogen activator modified in accordance with the present invention encompasses all such variant forms.

The modified t-PA of the invention may be derivatised to provide pharmaceutically useful conjugates analogous to known t-PA-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b)   an enzyme-protein conjugate as disclosed in EP-A-O 152 736, comprising at least one optionally blocked fibrinolytic enzyme linked by way of a site other than the catalytic site responsible for fibrinolytic activity to at least one human protein;

(c)   a protein-polymer conjugate as disclosed in EP-A-O 183 503 comprising a pharmaceutically useful protein linked to at least one water soluble polymer by means of a reversible linking group; or

(d)   an enzyme conjugate as disclosed in EP-A-O 184 363 comprising a plurality of fibrinolytic enzymes linked together through the active centres thereof by means of a removable blocking group.

The modified t-PA of the invention may take the place of t-PA as the enzyme or (human) protein component, as appropriate, of any of the conjugates described above.

The modified t-PA of the invention or conjugate thereof can be further derivatised such that any catalytic site essential for fibrinolytic activity is optionally blocked by a removable blocking group.

The above mentioned derivatives of the modified t-PA may be used in any of the methods and compositions described hereinafter for the modified t-PA itself.

As used herein the expression 'removable blocking group' includes groups which are removable by hydrolysis at a rate such that the pseudo-first order rate constant for hydrolysis is in the range of $10^{-6}$ sec$^{-1}$ to $10^{-2}$ sec$^{-1}$ in isotonic aqueous media at pH 7.4 at 37$^{\circ}$C.

- 5 -

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$ alkylphenyl.

In a further aspect, the invention provides a process for preparing modified tissue-type plasminogen activator according to the invention which process comprises expressing DNA encoding said modified tissue-type plasminogen activator in a recombinant host cell and recovering the modified tissue-type plasminogen activator product.

The modification of the t-PA can therefore be carried out by conventional genetic engineering techniques in which the cDNA which codes for t-PA is modified and the modified cDNA expressed in a prokaryote or eukaryote host.

The DNA polymer comprising a nucleotide sequence that encodes the modified t-PA also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

    i)    preparing a replicable expression vector capable, in a host cell, of expressing a DNA polymer comprising a nucleotide sequence that encodes said modified tissue-type plasminogen activator;

    ii)  transforming a host cell with said vector;

    iii) culturing said transformed host cell under conditions permitting expression of said DNA polymer to produce said modified tissue-type plasminogen activator; and

    iv)  recovering said modified tissue-type plasminogen activator.

The invention also provides a process for preparing the DNA polymer by the condensation of appropriate mono-, di- or oligomeric nucleotide units.

The preparation may be carried out chemically, enzymatically, or by a combination of the two methods, in vitro or in vivo as appropriate. Thus, the DNA polymer may be prepared by the enzymatic ligation of appropriate DNA fragments, by conventional methods such as those described by D. M. Roberts et al in Biochemistry 1985, 24, 5090-5098. The DNA fragments may be obtained by the digestion of DNA containing the required sequences of nucleotides with appropriate restriction enzymes, by chemical synthesis, by enzymatic polymerisation, or by a combination of these methods.

- 7 -

Digestion with restriction enzymes may be performed in an appropriate buffer at a temperature of $20^{\circ}$-$70^{\circ}$C, generally in a volume of $50\mu l$ or less with $0.1$-$10\mu g$ DNA.

Enzymatic polymerisation of DNA may be carried out _in vitro_ using a DNA polymerase such as DNA polymerase I (Klenow fragment) in an appropriate buffer containing the nucleoside triphosphates dATP, dCTP, dGTP and dTTP as required at a temperature of $10^{\circ}$-$37^{\circ}$C, generally in a volume of $50\mu l$ or less.

Enzymatic ligation of DNA fragments may be carried out using a DNA ligase such as T4 DNA ligase in an appropriate buffer at a temperature of $4^{\circ}$C to ambient, generally in a volume of $50\mu l$ or less.

The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

- 8 -

DNA polymer which encodes the modified t-PA may be prepared by site-directed mutagenesis of the cDNA which codes for tissue-type plasminogen activator, by conventional methods such as those described by G. Winter et al in Nature 1982, 299, 756-758 or by Zoller and Smith 1982; Nucl. Acids Res., 10, 6487-6500, or deletion mutagenesis such as described by Chan and Smith in Nucl. Acids Res., 1984, 12, 2407-2419 or by G. Winter et al in Biochem. Soc. Trans., 1984, 12, 224-225.

In particular, the preparation may be carried out by the simultaneous or sequential application of the mutagenesis methods disclosed in EP-A-0 207 589 and EP-A-0 201 153. Thus, the preparation may comprise the simultaneous mutagenesis of the cDNA coding for native t-PA in the region coding for the growth factor domain (to provide modification (a)) and at the codon encoding lysine 277 (to provide modification (b)). Alternatively, the preparation may comprise the mutagenesis of DNA which codes for t-PA having one of the modifications (a) and (b), to provide the other modification.

Alternatively and preferably, the DNA polymer may be prepared by the enzymatic ligation of a first DNA fragment which encodes a portion of t-PA encompassing modification (a) with a second DNA fragment which encodes a portion of t-PA encompassing modification (b). The DNA fragments may be obtained by the digestion of DNA polymers coding for t-PA containing modification (a) (disclosed in EP-A-0 207 589) and modification (b) (disclosed in EP-A-0 201 153) respectively. Conveniently the same restriction site is selected, lying between the sites or potential sites of both modifications, for both DNA polymers, so that

- 9 -

the resulting fragments may be ligated to restore the t-PA DNA sequence but including modifications (a) and (b). This site is preferably NarI.

The expression of the DNA polymer encoding the modified t-PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the modified t-PA, under ligating conditions.

Thus, the DNA polymer may be preformed or formed during the construction of the vector, as desired.

The ligation of the linear segment and more than one DNA molecule may be carried out simultaneously or sequentially as desired.

In one particular aspect, the replicable expression vector may be prepared by cleaving a vector compatible with the host cell to provide a linear DNA segment having an intact replicon, and combining said linear segment with the DNA polymer encoding the modified t-PA or fragments thereof, under ligating conditions.

The choice of vector will be determined in part by the host cell, which may be prokaryotic or eukaryotic.

Suitable vectors include plasmids, bacteriophages, cosmids and recombinant viruses.

The preparation of the replicable expression vector may be carried out conventionally with appropriate enzymes for restriction, polymerisation and ligation of the DNA, by procedures described in, for example, Maniatis et al cited above. Restriction, polymerisation and ligation may be performed as described above for the preparation of the DNA polymer.

The recombinant host cell is prepared, in accordance with the invention, by transforming a host cell with a replicable expression vector of the invention under transforming conditions. Suitable transforming conditions are conventional and are described in, for example, Maniatis et al cited above, or ''DNA Cloning'' Vol. II, D.M. Glover ed., IRL Press Ltd, 1985.

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]-propane-sulphonic acid, RbCl and glycerol. Mammalian cells in culture may be transformed by calcium co-precipitation of the vector DNA onto the cells.

The invention also extends to a host cell transformed with a replicable expression vector of the invention.

Culturing the transformed host cell under conditions permitting expression of the DNA polymer is carried out conventionally, as described in, for example, Maniatis et al and ''DNA Cloning'' cited above. Thus, preferably the cell is supplied with nutrient and cultured at a temperature below 45°C.

The modified t-PA expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium.

The DNA polymer may be assembled into vectors designed for isolation of stable transformed mammalian cell lines expressing the modified t-PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

It will be appreciated that, depending upon the host cell, the modified t-PA prepared in accordance with the invention may be glycosylated to varying degrees. Furthermore, as observed by Pohl et.al., Biochemistry, 1984, 23, 3701-3707, varying degress of glycosylation may also be found in unmodified, naturally occurring t-PA. The modified t-PA of the invention is understood to include such glycosylated variations.

The modified t-PA of the invention is suitably administered in the form of a pharmaceutical composition.

- 12 -

Accordingly the present invention also provides a pharmaceutical composition comprising modified t-PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the modified t-PA in solution and a local anaesthetic such as lignocaine to ease pain at the site of injection. Generally, the modified t-PA will be supplied in unit dosage form for example as a dry powder or water free concentrate in a hermetically sealed container such as an ampoule or sachette indicating the quantity of protein in activity units. Where the modified t-PA includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration. The quantity of material administered will depend upon the amount of fibrinolysis required and the speed with which it is required, the seriousness of the thromboembolic condition and position and size of the clot. The precise dose to be employed and mode of

- 13 -

administration must per force in view of the nature of the complaint be decided according to the circumstances by the physician supervising treatment. However, in general, a patient being treated for a mature, fresh or nascent thrombus will generally receive a daily dose of from 0.01 to 10 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified t-PA of the invention.

The invention also provides a modified t-PA of the invention for use as an active therapeutic substance and, in particular, for use in the treatment of thrombotic diseases.

The following Example illustrates the invention.

Methods used for Example 1
DNA cleavage

In general the cleavage of about 1µg of plasmid DNA or DNA fragments was effected using about 5 units of a restriction enzyme (or enzymes) in about 20µl of an appropriate buffer solution.

- 14 -

Ligation of DNA Fragments: Ligation reactions were carried out as described (Maniatis et al., Molecular Cloning - A Laboratory Manual, Cold Spring Harbor Laboratory 1982).

Transformation of plasmid DNA into E. coli HB101 cells was (a) as described by Hanahan (DNA Cloning Vol. I Chapter 6, D.M. Glover ed. IRL Press, 1985) in Protocol 3 except that incubation with RFI was for 5 minutes, (Example 1(a)), or (b) carried out using competent E. coli HB101 cells obtained from Gibco BRL (Paisley, Scotland) following the manufacturer's protocol, (Example 1(b)).

Plasmid preparation: Large scale preparation of plasmid DNA and plasmid mini-preparations were carried out as described in Maniatis et al., (Molecular Cloning - A Laboratory Manual, Cold Spring Harbor Laboratory, 1982).

Isolation of DNA fragments from low-melting-point (LMP) agarose gels

DNA fragments were isolated from LMP agarose gels as described by Maniatis et al., (Molecular Cloning - A Laboratory Manual, Cold Spring Harbor Laboratory 1982).

Dephosphorylation of DNA

Vector DNA was dephosphorylated, where appropriate, by treatment with calf intestinal alkaline phosphatase as described by Maniatis et al., (Molecular Cloning, A Laboratory Manual, Cold Spring Harbor Laboratory, 1982).

- 15 -

<u>Expression of mutant t-PA (Example 1(a))</u>

Cell preparation: cells were trypsinised and plated out at $10^6$ cells per 60mm dish and left overnight in growth medium (10% Serum, 1% stock solution of penicillin/streptomycin; Flow Laboratories, 1% Glutamine, 1% stock solution of non-essential amino acids; Flow Laboratories, in Eagles MEM) at 37°C in a humidified incubator in an atmosphere of 5% $CO_2$/95% air.

Transfection procedure: The transfection used (calcium coprecipitation) is described in 'DNA Cloning' Ed. D.M. Glover (Chap. 15, C. Gorman). Glycerol shock and 10mM butyrate treatment were used. Following transfection the cells were rested overnight in growth medium.

Overlay: Agarose (Indubiose $A_{37}$), 2.4g in 95ml Eagles MEM (Gibco) heated to melting point in a bunsen flame, was then placed in a water bath maintained at 48°C. 5.6 ml of fibrinogen (20mg/ml) were diluted 1:1 with 5.6 ml of Eagles MEM (containing an extra 7 mg/ml of NaCl) and retained on ice. 3.3 ml of Eagles MEM (no additions) were aliquoted into a bijou containing 86µl of bovine thrombin at 50 NIH Units/ml (retained on ice). The cells were washed 3 times with Eagles MEM to remove serum. The thrombin and fibrinogen were warmed to 37°C in a water bath. 9.5 ml agarose were aliquoted into a pre-warmed universal. The thrombin was added to the agar, followed by the fibrinogen. The gel was poured over the cell layer and incubated at 37°C until lysis zones appeared.

- 16 -

Expression of mutant t-PA (Example 1(b))

Cell preparation: cells were trypsinised and plated out at $5.4 \times 10^5$ cells per 60mm dish in growth medium (10% Serum (021-6010), 1% stock solution of penicillin/streptomycin (043-5070); 1% stock Glutamine (043-5030), 2% sodium bicarbonate (043-5080) in 1640 RPMI medium (041-2400); Gibco, Paisley, Scotland) at 37°C in a humidified incubator in an atmosphere of 5% $CO_2$/95% air. After 72h the cells were refed and were used for DNA transfection 24h later.

Transfection procedure: The transfection (in Eagles MEM) used calcium coprecipitation as described in 'DNA Cloning' Ed. D.M. Glover (Chap. 15, C. Gorman). Glycerol shock and 5mM butyrate treatment were used. Plasminogen activator secreted by transfected cells was harvested in medium (as above but serum-free, containing 4% soybean peptone (Sigma)) for 24h and activity was measured using fibrin plates with reference to normal t-PA (J.H. Robinson, Thromb. Res., 1984; 33, 155).

Example 1

Two mutated forms of t-PA have previously been described. The first carries an altered amino-acid at position 277 (numbering corresponds to that in Pennica et al., (1983) Nature 301, 214); an isoleucine in place of lysine (EP-A-0 201 153). The second has the growth factor domain deleted; amino acids 51-87 inclusive (EP-A-0 207 589). A third altered form of t-PA is described here which combines both the aforementioned mutations. The expression plasmid encoding this modified t-PA was constructed as follows. The approximately 900bp NarI-SstI fragment carrying the lys277 -> ile277 mutation was excised from pTRE18 (EP-A-0 201 153) and the approximately 410bp

- 17 -

HindIII-NarI fragment excised from plasmid pTRE24 (EP-A-0 207 589). These two fragments were ligated with the approx. 6kb HindIII-SstI fragment of pTRE15 (EP-A-0 207 589) and transformed into E. Coli HB101. Plasmid pTRE20 was isolated.

Example 1(a)

Plasmid pTRE20 prepared from recombinant E. coli (method (a)) by standard techniques was used to transfect cultured mouse L929 cells by the calcium phosphate precipitation technique essentially as described in 'High Efficiency Gene Transfer into Mammalian Cells' by C. Gorman (In 'DNA Cloning Volume II', ed. D. Glover, IRL Press Ltd., 1985).

Expression of modified and unmodified t-PA from transfected mouse L929 cells (24 hours post-transfection) was detected using the fibrin/agarose overlay technique as described in Methods section. Lytic zones were obtained.

Example 1(b)

Plasmid pTRE20 prepared from recombinant E. coli (method (b)) by standard techniques was used to transfect cultured human HeLa cells by the calcium phosphate precipi- tation technique ['High Efficiency Gene Transfer into Mammalian Cells' by C. Gorman (In 'DNA Cloning Volume II', ed. D. Glover, IRL Press Ltd., 1985) see Methods section].

Expression of modified and unmodified t-PA from transfected human HeLa cells was detected using the fibrin plate assay technique as described in Methods section. A sample of the harvest medium from cells transfected with the modified DNA was analysed by zymography as

- 18 -

described by Dodd et al., (1986), Thrombosis and Haemostasis 55(1); 94.

In the figure:

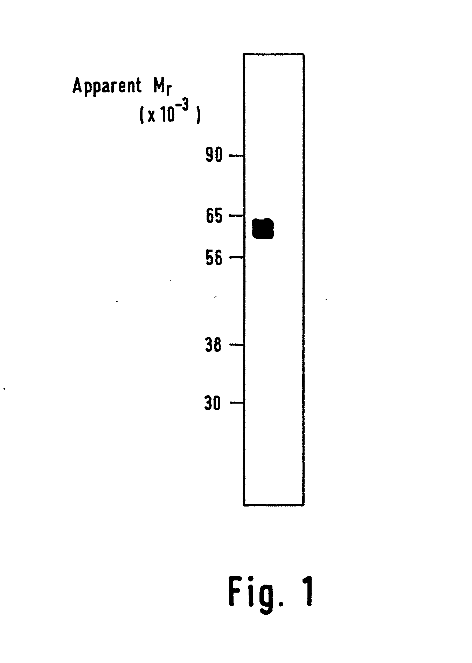

Figure 1    Fibrin Zymography of eukaryotic cell harvest medium

The modified t-PA had a mobility (apparent $M_r$) consistent with the predicted protein structure.

- 1 -

CLAIMS

1.    A fibrinolytically active tissue-type plasminogen activator which has been modified:

(a) in the region of the growth factor domain, and

(b) to provide an amino acid without a positively charged side chain in place of lysine at position 277.

2.    A modified tissue-type plasminogen activator according to claim 1, in which the modification (a) is in the region from amino acid residues 51 to 87 inclusive.

3.    A modified tissue-type plasminogen activator according to either of claims 1 or 2, wherein the modification (a) comprises the deletion of all or part of the growth factor domain.

4.    A modified tissue-type plasminogen activator according to claim 3, wherein the modification (a) comprises deletion of amino acid residues 51 to 87 inclusive.

5.    A modified tissue-type plasminogen activator according to any preceding claim, wherein the modification (b) provides an amino acid without a charged side chain in place of lysine at position 277.

6.    A modified tissue-type plasminogen activator according to any preceding claim, in which the amino acid without a positively charged side chain is isoleucine.

7. A modified tissue-type plasminogen activator prepared according to Example 1.

8. A derivative of the modified tissue-type plasminogen activator according to any of claims 1 to 7.

9. A DNA polymer comprising a nucleotide sequence that encodes a modified tissue-type plasminogen activator according to any of claims 1 to 7.

10. A replicable expression vector capable, in a host cell, of expressing a DNA polymer according to claim 9.

11. A host cell transformed with the vector according to claim 10.

12. A pharmaceutical composition comprising modified tissue-type plasminogen activator according to any of claims 1 to 8 in combination with a pharmaceutically acceptable carrier.

13. A modified tissue-type plasminogen activator according to any of claims 1 to 8 for use as an active therapeutic substance.

14. A modified tissue-type plasminogen activator according to any of claims 1 to 8 for use in the treatment of thrombotic disease.

15. Use of a modified tissue-type plasminogen activator according to any of claims 1 to 8 for the preparation of a medicament for the treatment of thrombotic disease.

16. A process for preparing modified tissue-type plasminogen activator according to claim 1, which process comprises expressing DNA encoding said modified tissue-type plasminogen activator in a recombinant host cell and recovering the modified tissue-type plasminogen activator product.

17. A process for preparing a DNA polymer according to claim 9, by the condensation of appropriate mono-, di- or oligomeric nucleotide units.

18. A process according to claim 17, which process comprises the enzymatic ligation of a first DNA fragment which encodes a portion of tissue-type plasminogen activator encompassing modification (a) with a second DNA fragment which encodes a portion of tissue-type plasminogen activator encompassing modification (b).

19. Modified tissue-type plasminogen activator obtainable by the process of claim 16.

B

CLAIMS for Spain

1.    A process for preparing a fibrinolytically active tissue-type plasminogen activator which has been modified:
(a)   in the region of the growth factor domain, and

(b)   to provide an amino acid without a positively charged side chain in place of lysine at position 277,

which process comprises expressing DNA encoding said modified tissue-type plasminogen activator in a recombinant host cell and recovering the modified tissue-type plasminogen activator product.

2.    A process according to claim 1, in which the modification (a) is in the region from amino acid residues 51 to 87 inclusive.

3.    A process according to claim 1 or 2, wherein the modification (a) comprises the deletion of all or part of the growth factor domain.

4.    A process according to claim 3, wherein the modification (a) comprises deletion of amino acid residues 51 to 87 inclusive.

5.    A process according to any preceding claim, wherein the modification (b) provides an amino acid without a charged side chain in place of lysine at position 277.

6.    A process according to any preceding claim, in which the amino acid without a positively charged side chain is isoleucine.

- 2 -

7. A process for preparing a DNA polymer comprising a nucleotide sequence that encodes a modified tissue-type plasminogen activator as defined in any of claims 1 to 6, by the condensation of appropriate mono-, di- or oligomeric nucleotide units.

8. A process according to claim 7, which process comprises the enzymatic ligation of a first DNA fragment which encodes a portion of tissue-type plasminogen activator encompassing modification (a) with a second DNA fragment which encodes a portion of tissue-type plasminogen activator encompassing modification (b).

9. A process for preparing a replicable expression vector capable, in a host cell, of expressing a DNA polymer as defined in claim 7, which process comprises cleaving a vector compatible with said host cell to provide a linear DNA segment having an intact replicon, and combining the said linear segment with one or more DNA molecules which, together with said linear segment, encode the modified tissue-type plasminogen activator, under ligating conditions.

10. A process for preparing a host cell transformed with a replicable expression vector as defined in claim 9, which process comprises transforming a host cell with said vector under transforming conditions.

Apparent $M_r$
$(\times 10^{-3})$

90 —

65 —
56 —

38 —

30 —

## Fig. 1

# EUROPEAN SEARCH REPORT

0241209

Application number

EP 87 30 2836

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | WO-A-8 601 538 (BIOGEN N.V.) * Abstract; page 4, line 25 - page 5, line 9; page 7, lines 3-21; page 9, lines 3-16; page 14, lines 15-21; claims * | 1 | C 12 N 15/00 C 12 N 9/64 A 61 K 37/54 |
| D,Y | PROCEEDINGS OF THE NATIONAL ACADEMY OF SCIENCES OF THE USA, vol. 81, no. 17, September 1984, pages 5355-5359, Washington, US; T. NY et al.: "The structure of the human tissue-type plasminogen activator gene: Correlation of intron and exon structures to functional and structural domains" * Introduction; page 5358, column 1, line 18 - page 5359, column 1, line 28 * | 1 | |
| Y | EP-A-0 093 619 (GENENTECH INC.) * Page 9, lines 19-33; page 10, lines 19-29; page 51, lines 16-24 * | 1 | |
| D,P Y | EP-A-0 201 153 (BEECHAM GROUP PLC) * Page 1, lines 13-35; page 6, lines 14-25; claims * | 1 | |

--- -/-

TECHNICAL FIELDS SEARCHED (Int Cl 4)

C 12 N

The present search report has been drawn up for all claims

| Place of search | Date of completion of the search | Examiner |
|---|---|---|
| THE HAGUE | 03-08-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,P Y | EP-A-0 207 589 (BEECHAM GROUP PLC) <br> * Page 1, lines 1-39; page 16, lines 20-36; claims * | 1 | |
| P,Y | EP-A-0 199 574 (GENENTECH INC.) <br> * Page 3, lines 4-31; page 5, lines 6-13; page 5, line 33 - page 6, line 27; page 9, line 33 - page 10, line 26; claims * | 1 | |
| A | PROCEEDINGS OF THE NATIONAL ACADEMY OF SCIENCES OF THE USA, vol. 80, no. 2, January 1983, pages 349-352, Washington, US; T. EDLUND et al.: "Isolation of cDNA sequences coding for a part of human tissue plasminogen activator" <br> * Pages 351,352: "Discussion" * | 1 | |
| D,A | FEBS LETTERS, vol. 163, no. 1, October 1983, pages 37-41, Elsevier Science Publishers Ltd, Amsterdam, NL; L. BANYAI et al.: "Common evolutionary origin of the fibrin-binding structures of fibronectin and tissue-type plasminogen activator" <br> * Introduction; page 38, column 2, lines 10-19; page 39, column 1, lines 3-8; figures 1A,2 * | 1 | TECHNICAL FIELDS SEARCHED (Int. Cl 4) |

The present search report has been drawn up for all claims

| Place of search | Date of completion of the search | Examiner |
|---|---|---|
| THE HAGUE | 03-08-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82